# EUROPÄISCHE PATENTSCHRIFT

(11) **EP 3 079 795 B1**
(45) Veröffentlichungstag und Bekanntmachung des Hinweises auf die Patenterteilung: **25.07.2018**
(21) Anmeldenummer: 14808945.1
(22) Anmeldetag: 03.12.2014
(51) Int. Cl.: C25C 3/02, C25B 1/26, C25B 15/08, B01D 53/62

(54) **SEQUESTRIERUNG VON KOHLENDIOXID DURCH BINDUNG ALS ALKALICARBONAT**
SEQUESTRATION OF CARBON DIOXIDE BY BINDING IT AS ALKALI CARBONATE
SÉQUESTRATION DE DIOXYDE DE CARBONE PAR LIAISON SOUS LA FORME DE CARBONATE ALCALIN

(30) Priorität: 10.12.2013 DE 102013225419
(43) Veröffentlichungstag der Anmeldung: 19.10.2016
(73) Patentinhaber: Siemens Aktiengesellschaft, 80333 München (DE)
(72) Erfinder: SCHMID, Günter, 91334 Hemhofen (DE); TAROATA, Dan, 91054 Erlangen (DE)
(86) Internationale Anmeldenummer: PCT/EP2014/076389
(87) Internationale Veröffentlichungsnummer: WO 2015/086394

(56) Entgegenhaltungen:
- WO-A2-2012/175368
- DE-A1-102009 007 755
- JP-A- 2002 014 195
- US-A1- 2010 077 752
- US-A1- 2011 033 355

## Beschreibung

Die vorliegende Erfindung betrifft ein Verfahren zur Herstellung von Chlor und Speicherung von Kohlenstoffdioxid, wobei eine Verbindung der Formel M⁺Cl⁻ elektrolytisch zu M und Cl₂ umgesetzt wird, wobei M⁺ = Na, K oder ein Gemisch davon ist, M mit Kohlenstoffdioxid und gegebenenfalls H₂O zu M₂CO₃ und/oder gegebenenfalls zu MHCO₃ umgesetzt wird, und M₂CO₃ und/oder gegebenenfalls MHCO₃ gespeichert wird, sowie eine Vorrichtung zur Durchführung eines solchen Verfahrens.

### Stand der Technik

Durch die Verbrennung von fossilen Brennstoffen wird momentan etwa 80% des weltweiten Energiebedarfs gedeckt. Durch diese Verbrennungsprozesse wurden im Jahr 2011 weltweit circa 34.032,7 Millionen Tonnen Kohlenstoffdioxid (CO₂) in die Atmosphäre emittiert. Diese Freisetzung ist der einfachste Weg, auch große Mengen an CO₂ (Braunkohlekraftwerke über 50000t pro Tag) zu entsorgen.

Das CO₂ kann daneben durch verschiedene Verfahren abgeschieden bzw. als Rohstoff genutzt werden.

Beispielsweise wird mit Hilfe von Monoethanolamin (MEA) das Kohlendioxid aus Rauchgas gewaschen. Anschließend wird der MEA-CO₂ Komplex bei erhöhter Temperatur gespalten. Reines CO₂ kann so beispielsweise in Gaskavernen oder oberirdischen Tanks gespeichert oder endgelagert werden. Auch nutzt die chemische Industrie CO₂, um beispielsweise Teile der Monomereinheiten in Polymeren durch Polycarbonat zu ersetzten. Bei dem so dargestellten Kunststoffschaum werden teilweise bessere Eigenschaften beobachtet als ohne die Carbonatzusätze. Auch sind aus DE102008031437-A1, WO2010000681-A2, WO2010000681-A3, EP2294643-A2, US20110113844-A1, CN102077395-A Verfahren bekannt, bei denen Alkalimetalle durch Reaktion mit CO₂ kraftwerkstaugliche thermische Energie und zugleich wertvolle Zwischenprodukte für die chemische Industrie liefern. Jedoch werden solche Verfahren nicht zwangsläufig alles produzierte Kohlendioxid abfangen können, und teilweise ist auch eine Nachrüstung bei bestehenden Anlagen zu teuer.

Weitere Verfeinerungen des Verfahrens sind aus WO2012/038330 und WO2013/156476 bekannt.

Die Diskussion über die negativen Auswirkungen des Treibhausgases CO₂ auf das Klima hat dazu geführt, dass über eine Wiederverwertung von CO₂ nachgedacht wird. Thermodynamisch gesehen liegt CO₂ sehr niedrig und kann daher nur schwer wieder zu brauchbaren Produkten reduziert werden. Deshalb wird über eine Endlagerung des CO₂s durch Einbringung in unterirdische Gaskavernen nachgedacht (Carbon Capture & Storage). Die Bevölkerung hat diese Form der CO₂-Endlagerung nicht akzeptiert, weil die Gefahr besteht, dass das erstickend wirkende Gas austritt und zu einer erheblichen Gesundheitsgefahr werden kann.

Es besteht somit ein Bedarf an einem Verfahren zur sicheren und effizienten Lagerung von Kohlendioxid.

### Zusammenfassung der Erfindung

Erfindungsgemäß wurde herausgefunden, dass eine solche Sequestrierung von Kohlendioxid auch durch Verwendung von Alkalimetallsalzen von Na und/oder K bei gleichzeitiger Erzeugung von Chlor möglich ist, wobei hier neben einer sicheren Lagerung von CO₂ zudem auch Wertprodukte gewonnen werden können. Vorteilhaft ist auch, dass NaCl und oder KCl auch auf einfache Weise und billig als Rohstoffe erhältlich sind.

Gemäß einem ersten Aspekt betrifft die vorliegende Erfindung ein Verfahren zur Herstellung von Chlor und Speicherung von Kohlenstoffdioxid, wobei
a) eine Verbindung der Formel M⁺Cl⁻ elektrolytisch zu M und Cl₂ umgesetzt wird, wobei M⁺ = Na, K oder ein Gemisch davon ist;
b) M mit Kohlenstoffdioxid und gegebenenfalls H₂O zu M₂CO₃ und/oder gegebenenfalls zu MHCO₃ umgesetzt wird; und
c) M₂CO₃ und/oder gegebenenfalls MHCO₃ gespeichert wird, wobei die Umsetzung von M mit Kohlenstoffdioxid und gegebenenfalls H₂O in Schritt b) durch Verbrennung von M in einer Atmosphäre, umfassend Kohlenstoffdioxid und gegebenenfalls H₂O, erfolgt.
Gemäß einem weiteren Aspekt betrifft die vorliegende Erfindung
eine Vorrichtung zur Herstellung von Chlor und Speicherung von Kohlenstoffdioxid, umfassend eine Elektrolyseeinrichtung E, die dazu ausgebildet ist, eine Verbindung der Formel M⁺Cl⁻ elektrolytisch zu M und Cl₂ umzusetzen, wobei M⁺ = Na, K oder ein Gemisch davon ist;
eine erste Zuführeinrichtung 1 für M⁺Cl⁻, die dazu ausgebildet ist, M⁺Cl⁻ der Elektrolyseeinrichtung E zuzuführen;
eine erste Abführeinrichtung 1' für Cl₂, die dazu ausgebildet ist, Cl₂ aus der Elektrolyseeinrichtung E zu entfernen;
eine zweite Abführeinrichtung 2' für M, die dazu ausgebildet ist, M aus der Elektrolyseeinrichtung E zu entfernen;
einen ersten Reaktor R zur Umsetzung von Kohlenstoffdioxid mit M, der dazu ausgebildet ist, M mit Kohlenstoffdioxid und gegebenenfalls H₂O zu M₂CO₃ und/oder gegebenenfalls zu MHCO₃ umzusetzen;
eine zweite Zuführeinrichtung 2 für M, die mit der zweiten Abführeinrichtung 2' für M verbunden und dazu ausgebildet ist, M dem ersten Reaktor R zuzuführen;
eine dritte Zuführeinrichtung 3 für Kohlenstoffdioxid und gegebenenfalls H₂O, die dazu ausgebildet ist, dem ersten Reaktor R Kohlenstoffdioxid und gegebenenfalls H₂O zuzuführen;
eine dritte Abführeinrichtung 3' für M₂CO₃ und/oder gegebenenfalls MHCO₃, die dazu ausgebildet ist, M₂CO₃ und/oder gegebenenfalls MHCO₃ aus dem ersten Reaktor R zu entfernen; und
eine Speichereinrichtung S zur Speicherung von M₂CO₃ und/oder gegebenenfalls MHCO₃, die dazu ausgebildet ist, M₂CO₃ und/oder gegebenenfalls MHCO₃, das aus der dritten Abführeinrichtung 3' kommt, zu speichern, wobei der erste Reaktor (R) einen Brenner zum Verbrennen von M mit Kohlenstoffdioxid und gegebenenfalls H₂O aufweist. Weitere Aspekte der vorliegenden Erfindung sind den abhängigen Ansprüchen und der detaillierten Beschreibung zu entnehmen.

### Beschreibung der Figuren

Die beiliegenden Zeichnungen sollen Ausführungsformen der vorliegenden Erfindung veranschaulichen und ein weiteres Verständnis dieser vermitteln. Im Zusammenhang mit der Beschreibung dienen sie der Erklärung von Konzepten und Prinzipien der Erfindung. Andere Ausführungsformen und viele der genannten Vorteile ergeben sich im Hinblick auf die Zeichnungen.
Die Elemente der Zeichnungen sind nicht notwendigerweise maßstabsgetreu zueinander dargestellt. Gleiche, funktionsgleiche und gleich wirkende Elemente, Merkmale und Komponenten sind in den Figuren der Zeichnungen, sofern nichts anderes ausgeführt ist, jeweils mit denselben Bezugszeichen versehen.
- Figur 1: zeigt schematisch eine erste beispielhafte Ausführungsform der vorliegenden Erfindung.
- Figur 2: zeigt schematisch eine zweite beispielhafte Ausführungsform der vorliegenden Erfindung.
- Figur 3: zeigt schematisch eine dritte beispielhafte Ausführungsform der vorliegenden Erfindung.

### Detaillierte Beschreibung der Erfindung

Die vorliegende Erfindung ist gemäß bestimmter Ausführungsformen auf ein Verfahren zur Herstellung von Chlor und Speicherung von Kohlenstoffdioxid gerichtet, wobei
a) eine Verbindung der Formel M⁺Cl⁻ elektrolytisch zu M und Cl₂ umgesetzt wird, wobei M⁺ = Na, K oder ein Gemisch davon ist;
b) M mit Kohlenstoffdioxid und gegebenenfalls H₂O zu M₂CO₃ und/oder gegebenenfalls zu MHCO₃ umgesetzt wird; und
c) M₂CO₃ und/oder gegebenenfalls MHCO₃ gespeichert wird.

Prinzipiell kann der Gesamtumsatz dieser Ausführungsform in folgender Gleichung dargestellt werden:

2 MCl + 2 CO₂ → M₂CO₃ + CO + Cl₂,

wobei M = Na und K bzw. deren Mischungen.

Es kann somit bei der Umsetzung in Schritt b) auch Kohlenstoffmonoxid erzeugt werden, welches dann zu weiteren chemischen Produkten umgesetzt wird.

Die CO₂-Speicherdichte pro eingesetztem Metallchlorid verdoppelt sich, wenn nicht das Carbonat, sondern das Hydrogencarbonat sequestriert wird.

2 MCl + 3 CO₂ + H₂O → 2 MHCO₃ + CO + Cl₂,

wobei M = Na und K bzw. deren Mischungen.

Bei der Umsetzung in Schritt c) sind beispielsweise folgende Reaktionen möglich, z.B. wenn Luft mit Sauerstoff-, Wasser- und Kohlendioxidanteil verwendet wird:

2 M + CO₂ → M₂O + CO

2 M + 2 CO₂ → M₂CO₃ + CO

2 M + ½ O₂ → M₂O; M₂O + CO₂ → M₂CO₃

2 M + 2 H₂O → MOH + H₂ MOH + CO₂ → MHCO₃

Das gemäß diesen Gleichungen gewonnene CO kann dem Verfahren nach Schritt b) entnommen werden, also aus dem Reaktor zur Umsetzung von Kohlenstoffdioxid mit M, und dient als Wertstoff. Es kann beispielsweise mit Wasserstoff in einem Fischer-Tropsch-Verfahren zu Alkoholen oder anderen längerkettigen Kohlenwasserstoff umgesetzt werden.

n CO + (2n+1) H₂ → CₙH₂ₙ₊₂ + n H₂O (Alkane)

n CO + (2n) H₂ → CₙH₂ₙ + n H₂O (Alkene)

n CO + (2n) H₂ → CₙH₂ₙ₊₁OH + (n-1) H₂O (Alkohole)

Die Reaktionen verlaufen exotherm, so dass keine zusätzliche Energie für den Prozess aufgewendet werden muss. Somit kann bei der Umsetzung in Schritt b) auch Kohlenstoffmonoxid erzeugt werden, welches zu weiteren chemischen Produkten umgesetzt werden kann. Diese Produkte stellen ihrerseits Wertstoffe dar, die als solche verkauft oder als Edukte für die chemische Industrie verwendet werden können.

Daneben könnte gegebenenfalls auch eine Umsetzung von M mit Stickstoff im Schritt b) erfolgen, so Stickstoff in der Atmosphäre der Umsetzung vorhanden ist. Hierbei könnten verschiedene Produkte von Stickstoff entstehen, welche dann weiter zu Ammoniak, Aminen (mit CO oder Alkanen, Alkenen, etc.), Nitroverbindungen, etc. reagieren können. Somit kann beispielsweise auch Ammoniak als Wertstoff gewonnen werden.

Zudem entsteht beim erfindungsgemäßen Verfahren Chlorgas, welches Schritt a) entnommen und gegebenenfalls gespeichert und/oder abtransportiert werden kann. Gemäß bestimmter Ausführungsformen kann es auch vor Ort für weitere chemische Reaktionen verwendet werden, beispielsweise zur Chlorierung der oben erzeugten Alkane, Alkene, Alkohole, etc., oder durch Umsetzung mit Wasserstoff zu HCl. Cl₂ dient der chemischen Industrie zur Herstellung von Lösungsmitteln, Intermediaten oder Salzsäure.

Die Jahresproduktion von Chlor beträgt etwa 71 Mio. t (Tonnen) in 2013. Dies entspricht bei dem erfindungsgemäßen Verfahren einer sequestrierbaren CO₂ Menge von 88 Mio. t. Zusätzlich kommen noch 44 Mio. t CO₂ hinzu, die des Gesamtprozess als CO (28 Mio. t) verlassen. Bezogen auf den Chlormarkt hat die erfindungsgemäße Prozessfolge somit ein Gesamt-CO₂-Volumen von 132 Mio. t/Jahr, die nicht in die Atmosphäre abgegeben werden. Bei einem CO₂-Zertifikatspreis von 30€/t(CO₂) entspricht dies einer Zertifikateinsparung von 3,96 Mrd. €. Bei einem niedrigeren voraussichtlichen CO₂ Emissionszertifikatspreis von ca. 7 bis 10 €/t CO₂ befindet sich alleine die Zertifikatseinsparung trotzdem bei ca. 1 Mrd. €. Aus 28 Mio. t CO lassen sich ca. 14 Mio. t Rohbenzin herstellen, die bei einem Marktwert von 650 €/t einem Gesamtvolumen von weiteren 9.1 Mrd. € entsprechen. Die 23 größten deutschen Braun- und Steinkohlekraftwerke stoßen ca. 200 Mio. t CO₂ aus. Dies bedeutet, dass 2/3 des CO₂-Abfalls deutscher Kraftwerke als Feststoff in der Erde endgelagert werden könnten, wenn die Prozessfolge etabliert wird und der Grundstoff Chlor gemäß des erfindungsgemäßen Prozessablaufs hergestellt wird.
Gemäß bevorzugten Ausführungsformen stammt die Verbindung der Formel M⁺Cl⁻ aus Abfallprodukten der chemischen Industrie und/oder wird aus ihnen gewonnen. Beispielsweise können solche (Abfall)-Produkte aus dem Salzabbau stammen.
Die Elektrolyse in Schritt a) ist erfindungsgemäß nicht besonders beschränkt und kann beispielsweise eine Schmelzflusselektrolyse einer Verbindung der Formel M⁺Cl⁻ oder eine Elektrolyse einer wässrigen Lösung der Verbindung der Formel M⁺Cl⁻ umfassen.
Bei Verwendung einer Schmelzflusselektrolyse eines Gemischs, umfassend eine Verbindung der Formel M⁺Cl⁻, ergibt sich der Vorteil, dass eine höhere Effizienz erzielt wird.

Andererseits kann, wenn abwechselnd eine Elektrolyse einer wässrigen Lösung der Verbindung der Formel M⁺Cl⁻ unter Entstehen von Wasserstoff erfolgt, dieser Wasserstoff beispielsweise als Wertprodukt verwendet werden oder gemäß bestimmten Ausführungsformen mit bei der Umsetzung in Schritt b) erzeugtem Kohlenstoffmonoxid zu weiteren chemischen Produkten umgesetzt werden, wie in den obigen Formeln beispielhaft veranschaulicht. Natürlich ist es auch nicht ausgeschlossen, dass Wasserstoff aus anderen Quellen für eine Umsetzung des in Schritt b) erzeugten Kohlenmonoxids stammt. Die Umsetzung des Kohlenmonoxids mit Wasserstoff kann beispielsweise auch im ersten Reaktor, aber auch in einem anderen Reaktor erfolgen.

Gemäß der Erfindung erfolgt die Umsetzung von M mit Kohlenstoffdioxid und gegebenenfalls H₂O in Schritt b) durch Verbrennung von M in einer Atmosphäre, umfassend Kohlenstoffdioxid und gegebenenfalls H₂O, Die Atmosphäre in Schritt b) ist jedoch nicht besonders beschränkt, insofern Kohlenstoffdioxid enthalten ist, und kann auch beispielsweise Luft oder Abluft aus einer Verbrennung, beispielsweise in herkömmlichen thermischen Verbrennungsprozessen wie in Kohlekraftwerken oder bei der Verbrennung von Erdöl und/oder Erdgas sein. Bevorzugt findet die Umsetzung in einer Atmosphäre statt, die im Vergleich zur normalen Umgebungsluft angereichert ist an Kohlenstoffdioxid, beispielsweise Abluft aus einer Verbrennung von kohlenstoffhaltigen Stoffen zur Erzeugung elektrischer Energie. Vorteilhaft bei einer Verbrennung in Schritt b) ist ein effizienter und rascher Reaktionsverlauf. Ggf. kann zu diesem Zweck die Verbrennung gestartet werden, beispielsweise durch Zugabe von Wasser oder durch elektrische oder andere Zündquellen wie Lichtbogen, Laser, etc.
In bestimmten Ausführungsformen kann aus der Umsetzung in Schritt b) zusätzliche thermische Energie gewonnen werden, die gegebenenfalls in elektrische Energie und/oder zur Vorwärmung von M verwendet werden kann. Auch kann die elektrische Energie zur Elektrolyse in Schritt a) verwendet werden. Gemäß bevorzugter Ausführungsformen wird die Energie zur elektrolytischen Umsetzung von M⁺Cl⁻ zu M und Cl₂ im Wesentlichen aus Überschussenergie aus erneuerbaren Energien bereitgestellt, also beispielsweise zu mehr als 50%, bevorzugt mehr als 70%, weiter bevorzugt mehr als 80% und besonders bevorzugt mehr als 90%, bezogen auf den Energiebedarf der Elektrolyse. Überschussenergie aus erneuerbaren Energien steht hierzu beispielsweise dann zur Verfügung, wenn mehr Strom durch erneuerbare und/oder herkömmliche Energiequellen zur Verfügung gestellt wird, als von den Verbrauchern abgenommen wird. Insbesondere ist hiermit die Energie gemeint, die im Überschuss durch erneuerbare Energiequellen wie Solaranlagen, Windanlagen, Wasserkraftanlagen, geothermische Anlagen, Biokraftanlagen (Biomasse) oder ähnlichem bereitstellen und die nicht lokal, regional und/oder überregional zum Zeitpunkt der Erzeuger von Verbrauchern abgenommen werden kann. Es ist hierbei nicht ausgeschlossen, dass Energie auch aus anderen Quellen bezogen wird, beispielsweise aus herkömmlichen Stromquellen und/oder aus der oben erzeugten Energie bei der Umsetzung in Schritt b). Gemäß besonders bevorzugten Ausführungsformen wird die Energie, die zur Elektrolyse der Verbindung der Formel M⁺Cl⁻ verwendet wird, zu 100% aus erneuerbaren Energiequellen bezogen, wobei zum Betreiben der Elektrolyseeinheit auch Energie, die nicht direkt mit der Elektrolyse der Verbindung der Formel M⁺Cl⁻ in Verbindung steht, wie beispielsweise zu Beleuchtungszwecken oder zum Betrieb von Pumpen, etc. auch aus anderen Energiequellen stammen kann, aber auch aus erneuerbaren Energiequellen.

Weiterhin umfasst ist durch die vorliegende Erfindung eine Vorrichtung, mit der das erfindungsgemäße Verfahren durchgeführt werden kann.

So betrifft die Erfindung gemäß bestimmten Ausführungsformen
eine Vorrichtung zur Herstellung von Chlor und Speicherung von Kohlenstoffdioxid, umfassend
eine Elektrolyseeinrichtung E, die dazu ausgebildet ist, eine Verbindung der Formel M⁺Cl⁻ elektrolytisch zu M und Cl₂ umzusetzen, wobei M⁺ = Na, K oder ein Gemisch davon ist;
eine erste Zuführeinrichtung 1 für M⁺Cl⁻, die dazu ausgebildet ist, M⁺Cl⁻ der Elektrolyseeinrichtung E zuzuführen;
eine erste Abführeinrichtung 1' für Cl₂, die dazu ausgebildet ist, Cl₂ aus der Elektrolyseeinrichtung E zu entfernen;
eine zweite Abführeinrichtung 2' für M, die dazu ausgebildet ist, M aus der Elektrolyseeinrichtung E zu entfernen;
einen ersten Reaktor R zur Umsetzung von Kohlenstoffdioxid mit M, der dazu ausgebildet ist, M mit Kohlenstoffdioxid und gegebenenfalls H₂O zu M₂CO₃ und/oder gegebenenfalls zu MHCO₃ umzusetzen;
eine zweite Zuführeinrichtung 2 für M, die mit der zweiten Abführeinrichtung 2' für M verbunden und dazu ausgebildet ist, M dem ersten Reaktor R zuzuführen;
eine dritte Zuführeinrichtung 3 für Kohlenstoffdioxid und gegebenenfalls H₂O, die dazu ausgebildet ist, dem ersten Reaktor R Kohlenstoffdioxid und gegebenenfalls H₂O zuzuführen;
eine dritte Abführeinrichtung 3' für M₂CO₃ und/oder gegebenenfalls MHCO₃, die dazu ausgebildet ist, M₂CO₃ und/oder gegebenenfalls MHCO₃ aus dem ersten Reaktor R zu entfernen; und
eine Speichereinrichtung S zur Speicherung von M₂CO₃ und/oder gegebenenfalls MHCO₃, die dazu ausgebildet ist, M₂CO₃ und/oder gegebenenfalls MHCO₃, das aus der dritten Abführeinrichtung 3' kommt, zu speichern.

Gemäß bestimmten Ausführungsformen kann die erfindungsgemäße Vorrichtung weiter eine Einrichtung zur Erzeugung von erneuerbarer Energie umfassen, die dazu ausgebildet ist, die Elektrolyseeinrichtung (E) mit elektrischer Energie zu versorgen. Die Einrichtung/Anlage zur Erzeugung erneuerbarer Energie ist hierbei nicht besonders beschränkt und kann beispielsweise Windkraftanlagen, Wasserkraftwerke, Geothermieanlagen, Solaranlagen, Gezeitenkraftwerke, biothermische Anlagen bzw. Biomasse-Anlagen, usw. umfassen.

Der erste Reaktor R kann in bestimmten Ausführungsformen einen Brenner zum Verbrennen von M mit Kohlenstoffdioxid und gegebenenfalls H₂O aufweisen. Zum Starten der Verbrennung können auch Zündquellen wie Lichtbogen zur Erzeugung eines Zündfunkens bzw. Plasmas vorhanden sein. Weitere bekannte Zündanlagen zur Erzeugung eines Zündfunkens bzw. Plasmas sind z.B. Magnetzünder, elektronische Zünder und Laserzünder.

Auch kann der erste Reaktor R gemäß bestimmter Ausführungsformen weitere Abführeinrichtungen für entstehende gasförmige Produkte wie CO, NH₃, etc. umfassen.

Gemäß bestimmten Ausführungsformen kann die Elektrolyseeinrichtung E eine Schmelzflusselektrolyseeinrichtung sein, oder gemäß weiteren bestimmten Ausführungsformen kann die Elektrolyseeinrichtung E derart ausgebildet sein, dass eine wässrige Lösung von M⁺Cl⁻ elektrolysiert wird, wobei die Vorrichtung dann auch weiter eine vierte Abführeinrichtung 4' für Wasserstoff umfassen kann, die dazu ausgebildet ist, Wasserstoff aus der Elektrolyseeinrichtung E zu entfernen.

Weiterhin kann die erfindungsgemäße Vorrichtung eine vierte Zuführeinrichtung 4 für Wasserstoff umfassen, die gemäß bestimmten Ausführungsformen mit der vierten Abführeinrichtung 4' für Wasserstoff verbunden und derart ausgebildet ist, dem ersten Reaktor R Wasserstoff zuzuführen. Natürlich kann auch eine vierte Zuführeinrichtung 4 für Wasserstoff vorhanden sein, die nicht mit der vierten Abführeinrichtung 4' für Wasserstoff verbunden ist, oder eine solche vierte Zuführeinrichtung 4 für Wasserstoff kann auch in Ausführungsformen vorhanden sein, bei denen die vierte Abführeinrichtung 4' für Wasserstoff nicht zwingend vorhanden ist, beispielsweise wenn die Elektrolyseeinrichtung E zur Durchführung einer Schmelzflusselektrolyse ausgestaltet ist.

Daneben kann die erfindungsgemäße Vorrichtung auch einen oder mehrere Speicher zur Speicherung von Chlor und/oder weiteren Produkten wie Kohlenmonoxid oder Wasserstoff und/oder auch Folgeprodukten wie COCl₂ (CO + Cl₂), HCl (Cl₂ + H₂), etc. umfassen, und/oder auch weitere Reaktoren zur Umsetzung von Kohlenmonoxid mit Wasserstoff und gegebenenfalls Speicher zur Speicherung von Produkten einer solchen Umsetzung von Kohlenmonoxid und Wasserstoff, beispielsweise Alkanen, Alkenen und/oder Alkoholen. Vorteilhaft ist hierbei, dass diese Produkte bei einer gleichzeitigen Sequestrierung von Kohlendioxid erzeugt werden können. Eventuell bei solchen weiteren Reaktionen erzeugtes Kohlendioxid kann in das erfindungsgemäße Verfahren rückgeführt werden oder aber auch in die Atmosphäre abgelassen werden, wenn für die Reaktion in Schritt b) Abluft mit einer höheren Konzentration an Kohlendioxid, beispielsweise aus einer Verbrennung von kohlenstoffhaltigen Verbindungen, verfügbar ist, oder wenn die Elektrolyseeinheit E und der erste Reaktor R räumlich weit auseinandergelegen sind.

Es ist erfindungsgemäß nicht ausgeschlossen, dass die Elektrolyseeinheit E und der erste Reaktor R sich an räumlich weit entfernten Orten befinden, etwa wenn die Elektrolyseeinheit E sich in der Nähe von Anlagen zur Erzeugung erneuerbarer Energie befindet und der erste Reaktor R sich an einem anderen Ort befindet, wo bereits zuvor eine Verbrennung von Kohlenstoffdioxid mit dem Metall M oder ähnliche Reaktionen durchgeführt wurden und ein Transport des Metalls M von der Elektrolyseeinrichtung E zum ersten Reaktor R, beispielsweise per Schiff, Bahn oder LKW, günstiger ist als einen neuen ersten Reaktor R nahe zur Elektrolyseeinheit E zu errichten, was mit erheblichen Kosten verbunden sein kann. Auch ist es nicht ausgeschlossen, dass der erste Reaktor R und die Speichereinrichtung S sich an räumlich weit entfernten Ort befinden, etwa wenn in der Nähe zum ersten Reaktor R nicht genügend Platz für eine Speicherung der erzeugten Produkte, wie etwa M₂CO₃ und/oder gegebenenfalls MHCO₃, vorhanden ist, oder wenn diese Produkte an anderer Stelle auch Abnehmer als Edukte finden und eine Lagerung vor Ort bei den Abnehmern bevorzugt ist.

Gemäß bestimmter Ausführungsformen befinden sich die Elektrolyseeinheit E, der erste Reaktor R und/oder die Speichereinrichtung S sowie gegebenenfalls weitere Speicher für weitere Produkte jedoch nicht voneinander zu weit entfernt, um die Erzeugung von Kohlenstoffdioxid durch einen Transport von etwa M, M₂CO₃ und/oder MHCO₃ oder anderen erzeugten Stoffen zu weiteren Speichern möglichst zu vermeiden. Auch befindet sich die Elektrolyseeinheit E bevorzugt in der Nähe von Anlagen zur Erzeugung erneuerbarer Energien, die häufig Überschussenergie produzieren, die nicht lokal oder regional abgenommen werden kann. Da dies jedoch auch saisonal schwanken kann, ist es auch möglich, dass unterschiedliche Elektrolyseeinrichtungen E zu unterschiedlichen Zeiten den ersten Reaktor R mit M versorgen.

Die verschiedenen Zuführeinrichtungen, die erste Zuführeinrichtung 1, die zweite Zuführeinrichtung 2, die dritte Zuführeinrichtung 3 und gegebenenfalls die vierte Zuführeinrichtung 4, sowie die verschiedenen Abführeinrichtungen, die erste Abführeinrichtung 1', die zweite Abführeinrichtung 2', die dritte Abführeinrichtung 3' und gegebenenfalls die vierte Abführeinrichtung 4' sind erfindungsgemäß nicht besonders beschränkt und kann (Einfüll-)Trichter, Rohre, Förderbänder, etc. umfassen, aber auch Transportmittel wie LKWs, Schiffe, Frachtcontainer auf Zügen, etc., beispielsweise bei einer Beförderung von M, welche geeignet vorgesehen werden können.

Nebenbei sei angemerkt, dass die Zwischenprodukte Alkalimetall M, Alkalilauge MOH, welche bei der Verbrennung von M mit Wasser entstehen kann, Alkalihydrogencarbonat oder Alkalicarbonat für sich selbst schon einen Wert besitzen und ggf. bisher auf anderem Wege erhalten wurden. Natriumcarbonat wird beispielsweise derzeit aus natürlichen Quellen gewonnen, und andere Produkte wie Natronlauge durch andere chemische Prozesse. Da das erfindungsgemäße Verfahren eine wesentlich größere Menge an Natriumcarbonat oder Natriumhydrogencarbonat und/oder Natronlauge liefert, kann es somit beispielsweise die Förderung solcher Stoffe aus natürlichen Quellen oder Herstellung durch andere Verfahren ggf. ersetzen oder zumindest vermindern.

Diese prozessbedingten Vorteile kommen insbesondere zu Stande, wenn eine wässrige Alkalimetallchloridelektrolyse, wobei das Alkalimetall K oder Na ist, durch eine Schmelzflusselektrolyse ersetzt wird und die Stoffe entsprechend der Prozessfolgen in reiner oder abgewandelter Form umgesetzt werden. Energetisch wird der Gesamtprozess in bestimmten Ausführungsformen insbesondere getrieben durch eine Überproduktion an elektrischer Energie, die nicht ins Netz eingespeist werden kann.

Prinzipiell kann das erfindungsgemäße Verfahren an Standorten eingesetzt werden, die jetzt die Elektrolyse von NaCl und oder KCl, beispielsweise bei einer wässrigen Natriumchloridlösung, nutzen, um den Rohstoff Chlor zu erhalten. Für den dabei entstehenden Wasserstoff gibt es bisher oft keine Verwendung, wobei er, wie oben gezeigt, zur Herstellung höherwertiger Produkte wie Alkane, Alkene oder Alkohole verwendet werden kann. Sollte zu viel Wasserstoff vorhanden sein, ist die Verwendung einer Schmelzflusselektrolyse von Vorteil, so dass je nach Vorhandensein und Nachfrage nach Wasserstoff beispielsweise auch abwechselnd eine Elektrolyse einer wässrigen Lösung von M⁺Cl⁻ oder eine Schmelzflusselektrolyse durchgeführt werden können, um entsprechend der Nachfrage und/oder dem Bedarf zur Herstellung höherwertiger Produkte, beispielsweise mit Fischer-Tropsch, Wasserstoff zur Verfügung zu haben.

Das Gesamtverfahren kann somit beispielsweise mit ggf. vorhandenen vorbereitenden Schritten wie folgt ablaufen:
0) Natriumchlorid und Kaliumchlorid finden sich in der Natur oft vergesellschaftet vor und werden relativ aufwändig durch elektrostatische Verfahren getrennt. Die Trennung ist jedoch nur bedingt effektiv, so dass große Kalium/Natriumchloridhalden entstehen, die aus Wirtschaftlichkeitsgründen nicht mehr weiter aufgearbeitet werden.

### Elektrostatisches Verfahren

Reibt man zum Beispiel einen Stoff wie die Salze NaCl bzw. KCl an einem anderen Material, so können sich beide gegeneinander "elektrisch" aufladen. Dieses Prinzip wird zur Trennung von Feststoffgemischen genutzt. Das Rohsalz wird hierzu beispielsweise in eine Korngröße von einem Millimeter aufgemahlen. Im nächsten Schritt können die Salze mit oberflächenaktiven Substanzen behandelt werden, so dass sie sich selektiv gegeneinander positiv und negativ aufladen. Danach riesein die Salzkristalle durch einen "Freifallscheider". Dieser besteht aus zwei Elektroden, zwischen denen ein elektrisches Hochspannungsfeld besteht. Die unterschiedlich geladenen Salze werden zur Anode bzw. zur Kathode abgelenkt. Unterhalb des Freifallscheiders werden die sortierten Mineralien getrennt aufgefangen.

Diese Halden können nun als Chloridquelle genutzt werden, wobei aber auch das Gemisch aus KCl und NaCl als solches als Chloridquelle in Schritt a) dienen kann. Weniger bevorzugt ist der direkte Abbau zu diesem Zweck. Steigen die CO₂-Zertifikate im Wert kann auch ein direkter Abbau wirtschaftlich werden. Vorzugsweise handelt es sich hierbei um eher wertlose Gemische mit unterschiedlichen Anteilen aus Kaliumchlorid und Natriumchlorid. Gemäß dem NaCl-KCl-Phasendiagramm werden die Schmelzpunkte der Salze in Gemischen deutlich herabgesetzt, was die Elektrolyse insbesondere im Falle einer Schmelzflusselektrolyse energetisch günstiger werden lässt. Typische Effizienzen sind hier beispielsweise größer als 50%. Für das Verfahren ist es unerheblich, ob die Alkalimetalle rein oder als Legierung der Einzelkomponenten abgeschieden werden. Weitere Salzzusätze (z.B. CaCl₂) zur Erniedrigung des Schmelzpunkts sind ebenfalls möglich.
1) Im zweiten Schritt werden aus den Halogeniden durch beispielsweise Schmelzflusselektrolyse die Alkalimetalle hergestellt, wie sie bereits bekannt ist.
   Einer Natriumelektrolyse nach dem Stand der Technik kann beispielsweise bei der Herstellung von Natrium durch Schmelzflusselektrolyse von trockenem Natriumchlorid in einer sogenannten Downs-Zelle erfolgen. Um eine effizientere Elektrolyse bei niedrigeren Temperaturen zu erreichen, kann hierbei zur Schmelzpunkterniedrigung beispielsweise ein eutektisches Salzgemisch aus 60 % Calciumchlorid und 40 % Natriumchlorid eingesetzt werden, das bei 580 °C schmilzt. Auch Bariumchlorid ist als Zusatz möglich. Nach Anlegen einer geeigneten Spannung, beispielsweise etwa sieben Volt, laufen beispielsweise die folgenden Prozesse ab:
   Kathode:

      2*Na*⁺+2*e⁻* → 2*Na*⁰
   Anode:

      *2Cl⁻* → *Cl*₂ + 2*e⁻*
   Gesamtreaktion:

      2*Na*⁺ + 2*Cl*⁻ → 2*Na* + *Cl*₂

   Beispielsweise werden für die Herstellung von einem Kilogramm Natrium während der Elektrolyse gemäß einem Verfahren des Stands der Technik etwa 10 kWh Strom verbraucht.
   Analoge Umsetzungen ergeben sich für KCl und/oder Gemische von NaCl und KCl.
2) Die Umsetzung in Schritt b) erfolgt dann beispielsweise gemäß den folgenden Gleichungen:

   2 M + CO₂ → M₂O + CO

   2 M + 2 CO₂ → M₂CO₃ + CO

   2 M + % O₂ → M₂O; M₂O + CO₂ → M₂CO₃

   2 M + 2 H₂O → MOH + H₂ MOH + CO₂ → MHCO₃

Bei der Verbrennung der Alkalimetalle in Atmosphären umfassend beispielsweise CO₂, N₂, O₂, H₂O können somit in der Vorrichtung bzw. im Kraftwerk umfassend den erster Reaktor R verwendbare Wärme, beispielsweise auch zum Vorheizen von M, und wertvolle Synthesebausteine wie CO, H₂ gewonnen werden.

Zur Erhöhung der Speicherdichte kann das Metallcarbonat mit Wasser und weiterem CO₂ zu Metallhydrogencarbonat umgesetzt werden:

M₂CO₃ + H₂O + CO₂ → 2 MHCO₃

Die Metallcarbonate bzw. Metallhydrogencarbonate können im Anschluss genutzt oder bei der erwarteten Überproduktion sequestriert werden.

Die obigen Ausführungsformen, Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmalen der Erfindung. Insbesondere wird der Fachmann auch Einzelaspekte als Verbesserungen oder Ergänzungen zu der jeweiligen Grundform der vorliegenden Erfindung hinzufügen.

### Beispiele

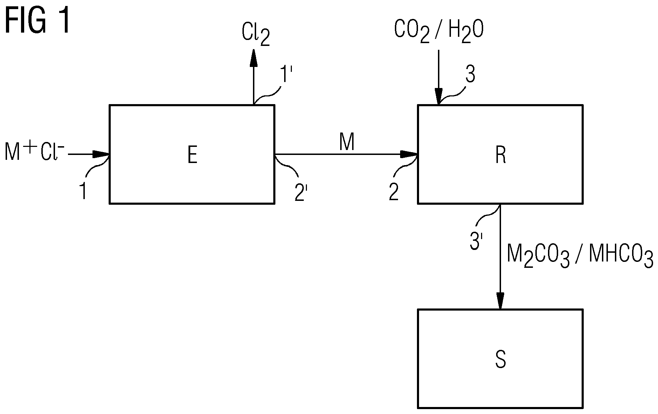

Beispielhafte Ausgestaltungen einer Anlage sind in den Figuren 1 bis 3 dargestellt.

### Beispiel 1

Eine erste Ausführungsform einer Vorrichtung gemäß der vorliegenden Erfindung ist in Figur 1 dargestellt.

Eine Verbindung M⁺Cl⁻ wird über eine erste Zuführeinrichtung 1 für M⁺Cl⁻ der Elektrolyseeinrichtung E zugeführt. Diese ist beispielsweise als Schmelzflusselektrolyse ausgeführt. In dieser wird die Verbindung dann zu Cl₂ und M elektrolysiert, wobei das Cl₂ der Elektrolyseeinrichtung E über eine erste Abführeinrichtung 1' für Cl₂ entnommen wird und danach gespeichert, abtransportiert oder weiter verwendet werden kann. Zudem wird M der Elektrolyseeinrichtung E über eine zweite Abführeinrichtung 2' für M entnommen. Diese ist mit einer zweiten Zuführeinrichtung 2 für M verbunden, durch welche M in den ersten Reaktor R zur Umsetzung von Kohlenstoffdioxid eingebracht wird. Weiterhin werden Kohlenstoffdioxid und gegebenenfalls H₂O über die dritte Zuführeinrichtung 3 für Kohlenstoffdioxid und gegebenenfalls H₂O dem ersten Reaktor R zugeführt. Dort wird M mit CO₂ und gegebenenfalls H₂O sowie gegebenenfalls weiteren Gasen wie N₂ oder O₂, welche nicht dargestellt sind, zu M₂CO₃ und/oder gegebenenfalls MHCO₃ sowie gegebenenfalls weiteren nicht dargestellten Produkten wie MOH, NH₃, etc. umgesetzt. Das erzeugte M₂CO₃ und/oder gegebenenfalls MHCO₃ werden dem ersten Reaktor R über eine dritte Abführeinrichtung 3' für M₂CO₃ und/oder gegebenenfalls MHCO₃ entnommen und zu einer Speichereinrichtung S zur Speicherung von M₂CO₃ und/oder gegebenenfalls MHCO₃ gebracht. Gegebenenfalls können M₂CO₃ und/oder gegebenenfalls MHCO₃ bei entsprechender Nachfrage der Speichereinrichtung S entnommen werden. Auch können die im ersten Reaktor R erzeugten gasförmigen Produkte dem ersten Reaktor R entnommen werden über eine nicht dargestellte Abführeinrichtung.

### Beispiel 2

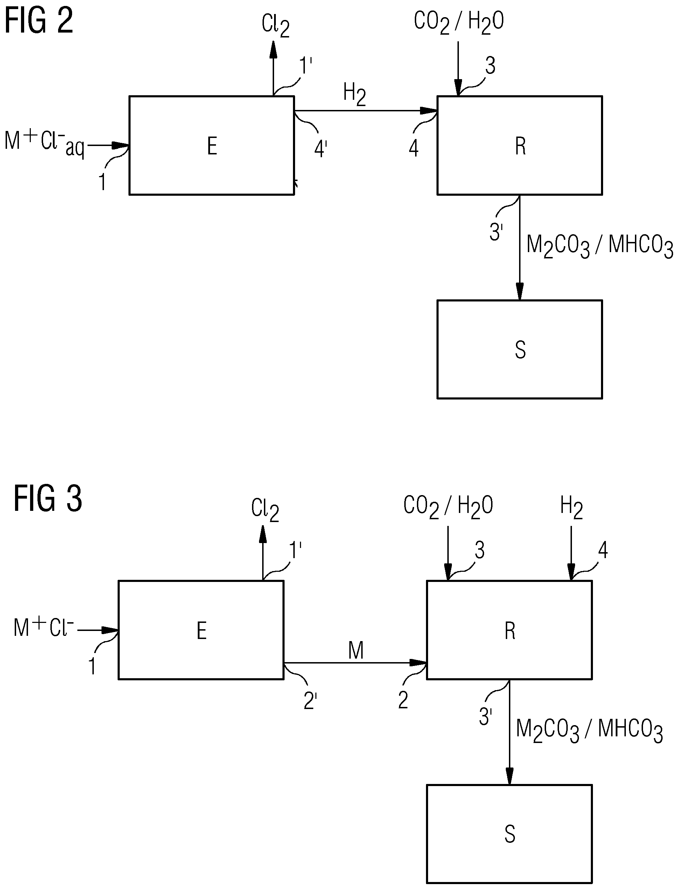

Die Vorrichtung von Beispiel 2 ist in Figur 2 dargestellt und entspricht der von Beispiel 1, wobei die Schmelzflusselektrolyse als Elektrolyseeinrichtung E durch eine solche ersetzt wird, in der eine wässrige Lösung vom M⁺Cl⁻ elektrolysiert wird. Dadurch entsteht in der Elektrolyseeinrichtung E Wasserstoff, der über eine vierte Abführeinrichtung 4' für Wasserstoff der Elektrolyseeinrichtung E entnommen wird. Diese vierte Abführeinrichtung 4' für Wasserstoff ist mit einer vierten Zuführeinrichtung 4 für Wasserstoff verbunden, mit der der Wasserstoff dem ersten Reaktor R zugeführt wird, wobei hier dann mit dem CO Alkane, Alkene, Alkohole etc. hergestellt werden können. Auch diese können über die nicht dargestellte Abführeinrichtung für gasförmige Produkte oder noch eine weitere Abführeinrichtung entnommen werden.

### Beispiel 3

Die in Figur 3 dargestellte Vorrichtung von Beispiel 3 entspricht der Vorrichtung in Beispiel 1, wobei dem ersten Reaktor R Wasserstoff über eine vierte Zuführeinrichtung 4 für Wasserstoff zugeführt wird. Wasserstoff kann wiederum mit CO zu Alkanen, Alkenen, Alkoholen, etc. umgesetzt werden, die über die nicht dargestellte Abführeinrichtung für gasförmige Produkte oder noch eine weitere Abführeinrichtung entnommen werden können.

Gemäß der vorliegenden Erfindung wird ein Gesamtkonzept beschrieben, das
a. in der Natur als Chloride vorkommende Stoffe wie Natriumchlorid oder Kaliumchlorid als Stoffquelle nutzt.
b. bevorzugt mit Hilfe von elektrischer Überschussenergie die Alkalimetalle Natrium und Kalium als stoffliche saisonale Energiespeicher durch Elektrolyse und Chlor für die Chlorproduktion erhält.
c. diese Metalle mit CO₂ zu Natriumcarbonat oder Kaliumcarbonat unter Bildung von CO verbrennt.
d. Natriumcarbonat oder Kaliumcarbonat bzw. -hydrogencarbonat sequestriert, womit CO₂ der Atomsphäre entzogen wird. Die sequestierbare Menge richtet sich ggf. nach der vorhandenen Überschussenergie und dem Chlorbedarf.
e. die chemische Industrie mit der Grundchemikalie Chlor zur Herstellung von Lösungsmitteln, Intermediaten oder Salzsäure versorgt.

Eine Kopplung der Chlorproduktion an die saisonalen stofflichen Energiespeicher Alkalimetalle eröffnet die Möglichkeit eines Prozesses zur Sequestrierung von CO₂ als Metallcarbonat oder Metallhydrogencarbonat.

Dieser Prozess ermöglicht zudem die Kopplung von "Carbon Capture and Storage" an die zeitlich und örtlich bedingte Überproduktion erneuerbarer Energien. Überschussenergie wird in Form von Alkalimetallen gespeichert. Die Entladung dieses Speichers in Verbindung mit CO₂ liefert thermische Energie auf hohem Temperaturniveau für die Rückverstromung und zusätzlich wertvolle Zwischenprodukte für die chemische Synthese.

Am Ende der Nutzungskette entsteht festes, leicht deponierbares ungiftiges Metallcarbonat und/oder Metallhydrogencarbonat.

Das maximal mögliche Volumen ist wohl limitiert auf den weltweiten Chlorbedarf, würde aber ausreichen, um 2/3 des CO₂-Ausstoßes der 24 größten deutschen Braun- und Kohlekraftwerke endzulagern.

Zusätzlicher finanzieller Benefit wird durch die Einsparung von CO₂-Zertifikaten und der Erhalt wertvoller Produkte wie Rohbenzin oder Alkohole erhalten.

## Patentansprüche

1. Verfahren zur Herstellung von Chlor und Speicherung von Kohlenstoffdioxid, wobei
a) eine Verbindung der Formel M⁺Cl⁻ elektrolytisch zu M und Cl₂ umgesetzt wird, wobei M⁺ = Na, K oder ein Gemisch davon ist;
b) M mit Kohlenstoffdioxid und gegebenenfalls H₂O zu M₂CO₃ und/oder gegebenenfalls zu MHCO₃ umgesetzt wird; und
c) M₂CO₃ und/oder gegebenenfalls MHCO₃ gespeichert wird, wobei die Umsetzung von M mit Kohlenstoffdioxid und gegebenenfalls H₂O in Schritt b) durch Verbrennung von M in einer Atmosphäre, umfassend Kohlenstoffdioxid und gegebenenfalls H₂O, erfolgt.

2. Verfahren gemäß Anspruch 1, wobei die Energie zur elektrolytischen Umsetzung von M⁺Cl⁻ zu M und Cl₂ im Wesentlichen aus Überschussenergie aus erneuerbaren Energien besteht.

3. Verfahren gemäß einem der vorgehenden Ansprüche, wobei die Verbindung der Formel M⁺Cl⁻ aus Abfallprodukten der chemischen Industrie gewonnen wird.

4. Verfahren gemäß einem der vorgehenden Ansprüche, wobei die Elektrolyse in Schritt a) durch Schmelzflusselektrolyse eines Gemischs, umfassend eine Verbindung der Formel M⁺Cl⁻, erfolgt.

5. Verfahren gemäß Anspruch 4, wobei bei der Umsetzung in Schritt b) auch Kohlenstoffmonoxid erzeugt wird, welches gegebenenfalls zu weiteren chemischen Produkten umgesetzt wird.

6. Verfahren gemäß einem der Ansprüche 1 bis 4, wobei abwechselnd eine Elektrolyse einer wässrigen Lösung der Verbindung der Formel M⁺Cl⁻ unter Entstehen von Wasserstoff erfolgt.

7. Verfahren gemäß Anspruch 6, wobei bei der Umsetzung in Schritt b) auch Kohlenstoffmonoxid erzeugt wird, welches mit dem Wasserstoff zu weiteren chemischen Produkten umgesetzt wird.

8. Vorrichtung zur Herstellung von Chlor und Speicherung von Kohlenstoffdioxid, umfassend
eine Elektrolyseeinrichtung (E), die dazu ausgebildet ist, eine Verbindung der Formel M⁺Cl⁻ elektrolytisch zu M und Cl₂ umzusetzen, wobei M⁺ = Na, K oder ein Gemisch davon ist;
eine erste Zuführeinrichtung (1) für M⁺Cl⁻, die dazu ausgebildet ist, M⁺Cl⁻ der Elektrolyseeinrichtung (E) zuzuführen;
eine erste Abführeinrichtung (1') für Cl₂, die dazu ausgebildet ist, Cl₂ aus der Elektrolyseeinrichtung (E) zu entfernen;
eine zweite Abführeinrichtung (2') für M, die dazu ausgebildet ist, M aus der Elektrolyseeinrichtung (E) zu entfernen;
einen ersten Reaktor (R) zur Umsetzung von Kohlenstoffdioxid mit M, der dazu ausgebildet ist, M mit Kohlenstoffdioxid und gegebenenfalls H₂O zu M₂CO₃ und/oder gegebenenfalls zu MHCO₃ umzusetzen;
eine zweite Zuführeinrichtung (2) für M, die mit der zweiten Abführeinrichtung (2') für M verbunden und dazu ausgebildet ist, M dem ersten Reaktor (R) zuzuführen;
eine dritte Zuführeinrichtung (3) für Kohlenstoffdioxid und gegebenenfalls H₂O, die dazu ausgebildet ist, dem ersten Reaktor (R) Kohlenstoffdioxid und gegebenenfalls H₂O zuzuführen;
eine dritte Abführeinrichtung (3') für M₂CO₃ und/oder gegebenenfalls MHCO₃, die dazu ausgebildet ist, M₂CO₃ und/oder gegebenenfalls MHCO₃ aus dem ersten Reaktor (R) zu entfernen; und
eine Speichereinrichtung (S) zur Speicherung von M₂CO₃ und/oder gegebenenfalls MHCO₃, die dazu ausgebildet ist, M₂CO₃ und/oder gegebenenfalls MHCO₃, das aus der dritten Abführeinrichtung (3') kommt, zu speichern, wobei der erste Reaktor (R) einen Brenner zum Verbrennen von M mit Kohlenstoffdioxid und gegebenenfalls H₂O aufweist.

9. Vorrichtung gemäß Anspruch 8, weiter umfassend eine Einrichtung zur Erzeugung von erneuerbarer Energie, die dazu ausgebildet ist, die Elektrolyseeinrichtung (E) mit elektrischer Energie zu versorgen.

10. Vorrichtung gemäß einem der vorgehenden vorrichtungsbezogenen Ansprüche, wobei die Elektrolyseeinrichtung (E) eine Schmelzflusselektrolyseeinrichtung ist.

11. Vorrichtung gemäß einem der Ansprüche 8 und 9, wobei die Elektrolyseeinrichtung (E) derart ausgebildet ist, dass abwechselnd eine wässrige Lösung von M⁺Cl⁻ elektrolysiert wird, weiter umfassend eine vierte Abführeinrichtung (4') für Wasserstoff, die dazu ausgebildet ist, Wasserstoff aus der Elektrolyseeinrichtung (E) zu entfernen.

12. Vorrichtung gemäß Anspruch 11, weiterhin umfassend eine vierte Zuführeinrichtung (4) für Wasserstoff, die mit der vierten Abführeinrichtung (4') für Wasserstoff verbunden und derart ausgebildet ist, dem ersten Reaktor (R) Wasserstoff zuzuführen.

## Claims

1. Method for producing chlorine and storing carbon dioxide, wherein
a) a compound of the formula M⁺Cl⁻ is reacted electrolytically to M and Cl₂, wherein M⁺ = Na, K or a mixture thereof;
b) M is reacted with carbon dioxide and optionally H₂O to M₂CO₃ and/or optionally to MHCO₃; and
c) M₂CO₃ and/or optionally MHCO₃ is stored,
wherein the reaction of M with carbon dioxide and optionally H₂O in step b) is carried out by burning M in an atmosphere comprising carbon dioxide and optionally H₂O.

2. Method according to Claim 1, wherein the energy for the electrolytic reaction of M⁺Cl⁻ to M and Cl₂ consists substantially of excess energy from renewable energies.

3. Method according to either of the preceding claims, wherein the compound of the formula M⁺Cl⁻ is obtained from waste products of the chemical industry.

4. Method according to one of the preceding claims, wherein the electrolysis in step a) is carried out by fused salt electrolysis of a mixture comprising a compound of the formula M⁺Cl⁻.

5. Method according to Claim 4, wherein in the reaction in step b) carbon monoxide is also formed, which is optionally reacted to give further chemical products.

6. Method according to one of Claims 1 to 4, wherein an electrolysis of an aqueous solution of the compound of the formula M⁺Cl⁻ with the formation of hydrogen is carried out alternately.

7. Method according to Claim 6, wherein in the reaction in step b) carbon monoxide is also produced, which is reacted with the hydrogen to give further chemical products.

8. Device for producing chlorine and storing carbon dioxide, comprising
an electrolysis device (E), which is designed to react a compound of the formula M⁺Cl⁻ electrolytically to M and Cl₂, wherein M⁺ = Na, K or a mixture thereof;
a first feed device (1) for M⁺Cl⁻, which is designed to feed M⁺Cl⁻ to the electrolysis device (E),
a first discharge device (1') for Cl₂, which is designed to remove Cl₂ from the electrolysis device (E);
a second discharge device (2') for M, which is designed to remove M from the electrolysis device (E);
a first reactor (R) for reacting carbon dioxide with M, which is designed to react M with carbon dioxide and optionally H₂O to M₂CO₃ and/or optionally to MHCO₃;
a second feed device (2) for M, which is connected to the second discharge device (2') for M and is designed to feed M to the first reactor (R);
a third feed device (3) for carbon dioxide and optionally H₂O, which is designed to feed carbon dioxide and optionally H₂O to the first reactor (R);
a third discharge device (3') for M₂CO₃ and/or optionally MHCO₃, which is designed to remove M₂CO₃ and/or optionally MHCO₃ from the first reactor (R); and
a storage device (S) for storing M₂CO₃ and/or optionally MHCO₃, which is designed to store M₂CO₃ and/or optionally MHCO₃ which comes from the third discharge device (3'),
wherein the first reactor (R) has a burner for burning M with carbon dioxide and optionally H₂O.

9. Device according to Claim 8, further comprising a device for generating renewable energy, which is designed to supply electrical energy to the electrolysis device (E).

10. Device according to one of the preceding device-related claims, wherein the electrolysis device (E) is a fused salt electrolysis device.

11. Device according to either of claims 8 and 9, wherein the electrolysis device (E) is so designed that an aqueous solution of M⁺Cl⁻ is electrolyzed alternately, further comprising a fourth discharge device (4') for hydrogen, which is designed to remove hydrogen from the electrolysis device (E).

12. Device according to claim 11, further comprising a fourth feed device (4) for hydrogen, which is connected to the fourth discharge device (4') for hydrogen and is designed to feed hydrogen to the first reactor (R).

## Revendications

1. Procédé de production de chlore et de stockage de dioxyde de carbone, dans lequel
a) on transforme un composé de formule M⁺Cl⁻ électrolytiquement en M et Cl₂, dans lequel M⁺ = Na, K ou l'un de leurs mélanges;
b) on fait réagir M sur du dioxyde de carbone et, le cas échéant, sur H₂O pour donner du M₂CO₃ et/ou, le cas échéant, du MHCO₃ et
c) on stocke M₂CO₃ et/ou, le cas échéant, MHCO₃, la réaction de M sur le dioxyde de carbone et, le cas échéant, sur H₂O au stade b) s'effectuant par combustion de M dans une atmosphère comprenant du dioxyde de carbone et, le cas échéant, de l'H₂O.

2. Procédé suivant la revendication 1, dans lequel l'énergie, pour la transformation électrolytique de M⁺Cl⁻ en M et Cl₂, est constituée essentiellement d'énergie en excès provenant d'énergies renouvelables.

3. Procédé suivant l'une des revendications précédentes, dans lequel on obtient le composé de formule M⁺Cl⁻ à partir de sous-produits de l'industrie chimique.

4. Procédé suivant l'une des revendications précédentes, dans lequel l'électrolyse au stade a) s'effectue par une électrolyse par fusion d'un mélange comprenant un composé de formule M⁺Cl⁻.

5. Procédé suivant la revendication 4, dans lequel, dans la réaction au stade b), on produit aussi du monoxyde de carbone, que l'on transforme, le cas échéant, en d'autres produits chimiques.

6. Procédé suivant l'une des revendications 1 à 4, dans lequel, en variante, on effectue une électrolyse d'une solution aqueuse du composé de formule M⁺Cl⁻ avec formation d'hydrogène.

7. Procédé suivant la revendication 6, dans lequel, dans la réaction au stade b), on produit aussi du monoxyde de carbone, que l'on fait réagir sur de l'hydrogène pour donner d'autres produits chimiques.

8. Installation de production de chlore et de stockage de dioxyde de carbone, comprenant
un dispositif (E) d'électrolyse constitué pour transformer un composé de formule M⁺Cl⁻ électrolytiquement en M et Cl₂, dans lequel M⁺ = Na, K ou l'un de leurs mélanges;
un premier dispositif (1) d'apport de M⁺Cl⁻, constitué pour apporter M⁺Cl⁻ au dispositif (E) d'électrolyse;
un premier dispositif (1') de sortie de Cl₂, constitué pour retirer Cl₂ du dispositif (E) d'électrolyse;
un deuxième dispositif (2') de sortie de M, constitué pour retirer M du dispositif (E) d'électrolyse;
un premier réacteur (R) pour faire réagir le dioxyde de carbone sur M, qui est constitué pour faire réagir M sur du dioxyde de carbone et, le cas échéant, sur de l'H₂O pour donner du M₂CO₃ et/ou, le cas échéant, du MHCO₃;
un deuxième dispositif (2) d'apport de M, qui communique avec le deuxième dispositif (2') de sortie et qui est constitué pour apporter M au premier réacteur (R);
un troisième dispositif (3) d'apport de dioxyde de carbone et, le cas échéant, d'H₂O, qui est constitué pour apporter du dioxyde carbone et le, cas échéant, de l'H₂O au premier réacteur (R) ;
un troisième dispositif (3') de sortie du M₂CO₃ et/ou, le cas échéant, de MHCO₃, qui est constitué pour retirer du M₂CO₃ et/ou, le cas échéant, du MHCO₃ du premier réacteur (R) et
un dispositif (S) de stockage pour stocker du M₂CO₃ et/ou, le cas échéant, du MHCO₃, qui est constitué pour stocker du M₂CO₃ et/ou, le cas échéant, du MHCO₃ venant du troisième dispositif (3') de sortie,
dans laquelle le premier réacteur (R) a un brûleur pour la combustion de M avec du dioxyde de carbone et, le cas échéant, de l'H₂O.

9. Installation suivant la revendication 8, comprenant, en outre, un dispositif de production d'énergie renouvelable, qui est constitué pour alimenter le dispositif (E) d'électrolyse en énergie électrique.

10. Installation suivant l'une des revendications précédentes se rapportant à une installation, dans laquelle le dispositif (E) d'électrolyse est un dispositif d'électrolyse de fusion.

11. Installation suivant l'une des revendications 8 et 9, dans laquelle le dispositif (E) d'électrolyse est constitué de manière, en variante, à électrolyser une solution aqueuse de M⁺Cl⁻ comprenant, en outre, un quatrième dispositif (4') de sortie d'hydrogène constitué pour retirer de l'hydrogène du dispositif (E) d'électrolyse.

12. Installation suivant la revendication 11, comprenant, en outre, un quatrième dispositif (4) d'apport d'hydrogène, qui communique avec le quatrième dispositif (4') de sortie d'hydrogène et qui est constitué de manière à apporter de l'hydrogène au premier réacteur (R).
